Europäisches Patentamt

European Patent Office

Office européen des brevets

(19)

(11) **EP 0 426 768 B1**

(12) **EUROPEAN PATENT SPECIFICATION**

(45) Date of publication and mention
of the grant of the patent:
**26.02.1997  Bulletin 1997/09**

(21) Application number: 89912918.3

(22) Date of filing: 14.11.1989

(51) Int Cl.$^6$: **C12Q 1/60**, C12Q 1/44,
C12Q 1/26, C12N 15/09,
C12N 9/02, C12N 1/21

(86) International application number:
**PCT/US89/05112**

(87) International publication number:
**WO 90/05788 (31.05.1990 Gazette 1990/12)**

(54) **Expression of a DNA sequence derived from a Nocardioform microorganism and encoding a cholesterol oxidase in a host Streptomyces microorganism**

Expression einer für eine Cholesterin-Oxydase kodierende DNS-Sequenz eines
Nocardioform-Mikroorganismus in einem Mikroorganismus der Sreptomyces-Gattung

Expression d'une sequence d'ADN dérivée d'un microorganisme du groupe des Nocardioformes et
codant pour une cholestérol oxydase dans un microorganisme-hôte du genre Streptomyces

(84) Designated Contracting States:
**AT BE CH DE FR GB IT LI LU NL SE**

(30) Priority: **14.11.1988  US 269669**

(43) Date of publication of application:
**15.05.1991  Bulletin 1991/20**

(73) Proprietor: **GENZYME CORPORATION
Boston Massachusetts 02111 (US)**

(72) Inventors:
• **LONG, Susan
Wakefield, MA 01880 (US)**
• **OSTROFF, Gary, R.
Needham, MA 02192 (US)**

(74) Representative: **Deans, Michael John Percy et al
Lloyd Wise, Tregear & Co.,
Commonwealth House,
1-19 New Oxford Street
London WC1A 1LW (GB)**

(56) References cited:
**EP-A- 0 035 480**          **US-A- 3 907 645**
**US-A- 4 409 326**

• **CHEMICAL ABSTRACTS, vol. 112, no. 13, 26
March 1990, Columbus, OH (US); O. IKEHATA et
al., p. 176, no. 112972f**
• **BIOLOGICAL ABSTRACTS, vol. 85, no. 6, 1988,
Philadelphia, PA (US); B. PETERLIN, p. 411, no.
57233**
• **JOURNAL OF BACTERIOLOGY, vol. 170,
February 1988; M.E. VOGT SINGER, pp. 638-645**
• **APPLIED & ENVIRONMENTAL MICROBIOLOGY,
vol. 52, December 1986; Y. MUROOKA, pp.
1382-1385**
• **American Type Culture Collection Catalogue of
bacteria and bacteriophages, 17th Edition, 1989,
pages 185, 186 and 270**
• **American Type Culture Collection Catalogue of
Recombinant DNA Materials, page 59**
• **The National Collection of Industrial Bacteria
Catalogue of Strains, 4th Edition, 1986**

**Description**

**Background of the Invention**

This invention relates to expression of a DNA sequence derived from a Nocardioform microorganism and encoding a cholesterol oxidase, in a host Streptomyces microorganism.

The organisms grouped as Nocardioforms in Bergey's Manual of Systematic Bacteriology (Williams and Wilkins, 1986, pp. 1458-1481) include nine genera which are close phylogenetically to Corynebacterium, Athrobacter and Mycobacterium. Members of the Nocardioforms including the genera Nocardia and Rhodococcus exhibit a wide range of useful metabolic activities including the assimilation of unusual compounds such as alkanes and aromatic hydrocarbons; the degradation of lignin, detergents and pesticides; the production of enzymes useful in xenobiotic transformations; and the biosynthesis of antibiotics, amino acids and biosurfactants. In particular, this group of bacteria have been exploited for their production of important steroid modifying enzymes, including cholesterol esterase and cholesterol oxidase, which are used in diagnostic assays to determine cholesterol levels in blood and serum.

The capacity to degrade cholesterol is widespread among microorganisms other than the Nocardioforms. For example, cholesterol oxidases have also been isolated from species of Mycobacterium, Pseudomonas, and Streptomyces. It is well known from scientific and patent literature and from commercial practice that the Nocardioform-type cholesterol oxidase is distinct from the other microbial cholesterol oxidases.

The Nocardioform enzyme is very stable and active over a wide pH range (pH 6.0 - 8.0), the Km for cholesterol is $1.4 \times 10^{-5}$ mol/liter at 25°, and the enzyme is highly specific for $\Delta^4$- or $\Delta^5$-3β-sterols. As produced in the Nocardioforms, cholesterol oxidase is membrane associated and requires detergent extraction and lipid remove for purification.

Singer, et al., J. Bacteriol., 1988, Vol. 170, pp. 638-645, describes expression of genes in Rhodococcus sp. from both E. coli and Streptomyces sp. and the development of a shuttle plasmid based on an E. coli plasmid and containing a Rhodococcus plasmid-derived origin of replication. The plasmid is capable of replicating in E. coli and in several but not all of the Rhodococcus species tested.

Murooka et al, *Applied and Environmental Microbiology,* vol. 52, 1986, pp. 1382-1385 describes cloning and expression of a *Steptomyces* cholesterol oxidase gene in *Streptomyces lividans* with plasmid pIJ702.

Summary of the Invention

We have discovered that recombinant DNA sequences derived from Nocardioform microorganisms can be expressed efficiently in a host microorganism.

The invention features, in one aspect, a cloning vector comprising a DNA sequence derived from a Nocardioform microorganism, said DNA sequence encoding a cholesterol oxidase, and said cloning vector being capable of expressing said DNA sequence in a host Streptomyces microorganism. In preferred embodiments, the DNA sequence is derived from Rhodococcus sp. NCIB 10554 (National Collection of Industrial Bacteria, Aberdeen, Scotland) and the host microorganism is S. lividans; and the cloning vector is the plasmid pSL81.

Cholesterol oxidase is produced according to the invention by providing a cloning vector containing a DNA sequence encoding cholesterol oxidase, which is derived from a Nocardioform microorganism, transforming a Streptomyces host cell with the cloning vector to obtain a recombinant host cell, culturing the recombinant host cell under conditions permitting expression of the DNA sequence, and recovering the cholesterol oxidase. In preferred embodiments, the Nocardioform organism is Rhodococcus sp. NCIB 10554; the host cell is preferably S. lividans; and the cloning vector is the plasmid pSL81.

Cholesterol oxidase produced according to the invention can be used for determining cholesterol in a sample, by contacting the sample with the recombinant cholesterol oxidase and determining the extent of oxidation of cholesterol; specifically, by measuring oxygen consumption with an oxygen electrode; or by measuring the production of hydrogen peroxide; or by spectrophotometrically determining cholesterol conversion to $\Delta^4$-cholestenone.

The invention provides for the expression of DNA sequences derived from a Nocardioform microorganism in a host microorganism that is easier to manipulate under conditions of commercial production than is the parent organism itself, and for which cloning techniques are known. Recombinant cholesterol oxidase derived from Nocardioform microorganisms is expressed as an extracellular protein, an expression condition which can lead to substantially higher production of enzyme. Moreover, the expression does not require addition of an inducer, and the expressed enzyme is free of the lipids of the Nocardioform membrane.

Other features and advantages of the invention will be apparent from the following description of the preferred embodiment thereof, and from the claims.

Description of the Preferred Embodiment

The drawing will first be described.

Drawing

Fig. 1 is a restriction endonuclease map of plasmid pSL81.
Fig. 2 (2.1-2.12) is a sequence description of the Rhodococcus 10554 cholesterol oxidase gene and corresponding amino acid sequence.
Fig. 3 is a restriction endonuclease map of plasmid pSL81, showing the 2.8 kb Bgl II fragment of Fig. 1.

Gene Isolation and Expression

The expression of DNA sequences derived from a Nocardioform microorganism can be accomplished, in general, by first constructing a genomic bank or library by incorporating the DNA sequences into a suitable cloning vector containing a suitable origin of replication compatible with the desired host organism. Next, a host cell from a species of Streptomyces is transformed with the vector containing the DNA sequences. Then, transformants are screened for the activity of the desired gene product, and active clones are isolated and characterized.

Construction of Library

The following protocol, presented for illustrative purposes, describes construction of a genomic library of Rhodococcus sp. NCIB 10554 (Rhodococcus 10554) DNA in a host such as S. lividans which is capable of expressing the DNA from the parent microorganism. In the example described below, a population of transformed cells prepared using this library is screened for the expression of the cholesterol oxidase gene of Rhodococcus 10554.
Generally, a genomic library cf Rhodococcus 10554 DNA was constructed from high molecular weight DNA which had been isolated and fragmented with the restriction enzyme Sau3A to yield fragments in the size range 4-8 kilobase pairs. Size fractionated DNA. from Rhodococcus 10554 was then ligated to the cloning plasmid pIJ702 which had been digested with BglII. The pIJ702 plasmid is commercially available through the American Type Culture Collection (Rockville, MD, USA)(ATCC No.35287 Hybrid plasmids of the library were transformed into S. lividans cells to generate the genomic library of Rhodococcus 10554 DNA in the host microorganism.
Details of the prccedure were as follows: A single colony of Rhodococcus 10554 was used to inoculate 50 ml of NBYE medium which contained per liter: Difco nutrient broth, 8g, and Difco yeast extract, 5g. Other appropriate media described for growing Rhodococcus sp. could also be used. The culture was grown for 48 hours at 30°c and the cells collected by centrifugation at 10,000 rpm fcr 15 minutes in a Sorvall RC-5B centrifuge using an SS-34 rotor. The cells were resuspended in 18 ml of Tris-glucose (10mM Tris-HCl, pH 8.0, 10mM EDTA, 25% w/v glucose), and 2 ml of lysozyme solution (10 mg/ml lysozyme (Sigma) in Tris-glucose solution) was added. This mixture was incubated at 37°C for 60 minutes. Next, 2.4 ml of 10% w/v sodium dodecyl sulfate (Sigma) was added and the mixture stirred vigorously to form a homogenous solution of lysed cells. The lysed cells were incubated at 55°C for 2 hours, and then 2.7 ml of 5M NaClO$_4$ (Sigma) were added. Denatured protein and cell debris were removed by extracting the lysed cell solution twice with 20 ml chloroform:isoamyl alcohol (24:1) and resolving the phases by centrifugation at 12,000 rpm for 10 minutes in a Sorvall SS-34 rotor. The aqueous phase was removed and two volumes of ccld ethanol added. The solution was mixed gently, and the high molecular weight DNA was collected using a glass rod as described by Marmur et al., 1961. J. Mol. Biol., Vol 3, pp. 203-218. The collected DNA was washed twice in 70% ethanol and dissolved in 5 ml TE (10mM Tris, 1mm EDTA, pH 8.0). DNAse free RNAse (Sigma) was added at 100 mg/ml to the DNA solution and incubated at 42°C for 30 minutes. Next, 0.6 ml of 10% w/v of sodium dodecyl sulfate and 0.6 ml of 5M NaCl were added, and the mixture was extracted once with phenol:chloroform (1:1) and once with chloroform:isoamyl alcohol (24:1). DNA was precipitated with 2 volumes of ethanol and collected as before using a glass rod. The DNA was washed once with 70% ethanol and air dried prior to resuspension in TE to a final concentration of approximately 1 mg/ml.
In order to establish the condition for isolating partially digested high molecular weight Rhodococcus 10554 DNA, test reactions were set up to digest DNA with different concentrations of Sau3A restriction enzyme. A reaction mixture was prepared with 10 μl DNA, 15 μl 10x Sau3A buffer (200mM Tris HCl, pH 7.4, 50mM MgCl$_2$, 500mM KCl) and 125 μl water. Aliquots of 15 μl were dispensed into tubes labeled 1-9 and chilled on ice. Exactly 4 units of Sau3A enzyme (10 units/μl BRL) were added to tube 1 and mixed well. Two-fold serial dilutions of the reaction mixtures from tube 1 sequentially through to tube 8 were performed to yield reaction mixtures containing two-fold decreasing concentrations of Sau3A enzyme. Tube 9 which contained no enzyme represented the undigested control reaction. The tubes were incubated at 37°C for 1 hour and stopped by returning the tubes to ice and adding EDTA (20mM). The extent of digestion in each tube was monitored by agarose gel electrophoresis, and the sample which yielded DNA fragments in the approximate range of 2-20 kb was scaled up 200 fold and the reaction repeated. The digested Rhodococcus 10554 DNA was separated by agarose gel electrophoresis using low gelling temperature agarose (SeaPlaque, FMC). The region of the gel containing DNA fragments in the size range 4-8 kb was excised with a sterile scalpel and the DNA recovered by electro-elution. The DNA was precipitated by conventional methods, and the precipitate was resuspended in TE to a concentration of 0.025 mg/ml.

A single colony of S. lividans/pIJ702 was used to inoculate 50 ml of YEME which contained per liter: Difco yeast extract, 3g; Difco peptone, 5g; Oxoid malt extract, 3g; glucose, 10g; sucrose, 340g; $MgCl_2$ at 5mM; and glycine at 0.4% w/v; and was supplemented with the plasmid selective agent thiostrepton (CalBiochem), at 50 µg/ml. Other media described for Streptomyces could also be used. The culture was incubated at 30°C for 3 days. The cells were harvested by centrifugation at 10,000 rpm for 30 minutes using a Sorvall centrifuge and SS-34 rotor. The pellet was resuspended in 5ml TSE Buffer (25mM Tris, pH 8.0; 0.3M sucrose; 25mM EDTA) containing 10 mg lysozyme. The cells were incubated at 37°C for 30 minutes. A 3 ml volume of alkaline SDS solution (0.3M NaOH, 2% w/v sodium dodecyl sulfate) was added, and the mixture was incubated at room temperature for 10 minutes and at 70°C for 10 minutes. The lysed cell solution was cooled to room temperature and extracted with 1 volume of phenol: chloroform (1:1). The aqueous phase was removed to a clean tube and 0.3M sodium acetate, pH 4.8, and 1 volume of isopropanol added. The DNA was precipitated for 30 minutes at -20°C and recovered by centrifugation, using a Sorvall SS-34 rotor at 10,000 rpm for 10 minutes. The pellet was resuspended in 2 ml TE and the plasmid DNA purified by ultracentrifugation to equilibrium in a cesium chloride density gradient according to known techniques.

Plasmid pIJ702 prepared as above was resuspended in TE to a concentration of 1 mg/ml. About 10 µl (10 µg) of DNA was added to 5 µl 10x BglII buffer (500mM Tris HCl, pH 7.4; 50mM $MgCl_2$; 500mM KCl), 32 µl distilled water and 3 µl of BglII restriction enzyme (10 units/µl, BRL) and incubated for 2 hours at 37°C. The reaction was terminated by one extraction with phenol:chloroform (1:1) and one extraction with chloroform:isoamyl alcohol (24:1). The DNA was precipitated by the addition of one-half volume of 7.5M ammonium acetate and 2 volumes of cold ethanol. After incubation at -20°C for at least 2 hours, the DNA was collected in an Eppendorf microfuge by centrifugation for 10 minutes. The DNA pellet was washed once with 70% ethanol, dried and resuspended in 50 µl 10mM Tris HCl, pH 8.0. To dephosphorylate the DNA, 1 µl of calf intestine alkaline phosphatase (2 U/µl, IBI) was added and the reaction incubated at 37°C for 30 minutes. The reaction was stopped by adding 150 µl stop buffer (10mM Tris HCl, pH 7.5; 1mm EDTA, pH 7.5; 200mM NaCl; 0.5% w/vsodium dodecyl sulfate) and then extracted once with phenol:chloroform (1:1) and once with chloroform:isoamyl alcohol (24:1). The DNA was precipitated with ethanol, dried and resuspended in TE to a final concentration of 0.05 mg/ml and stored at -20°C.

About 10 µl (0.5 µg) of the dephosphorylated BglII digested pIJ702 DNA was added to about 20 µl (0.5 pg) of the Sau3A digested Rhodococcus DNA. A 20 µl aliquot of 5x ligase buffer (BRL), 0.5 µl ligase (NEB) and 50 µl water were added and the ligation reaction incubated at 14°C for 20 hours. The reaction was terminated by heating the mixture to 65°C for 10 minutes, and the sample was stored at -20°C.

A vegetative inoculum was prepared by growing S. lividans in 25 ml culture broth as described for the preparation of S. lividans/pIJ702, except the medium contained no thiostrepton. The cells were harvested in a benchtop centrifuge at 3,000 rpm for 10 minutes and washed twice with 10 ml of 10.3% w/v sucrose. The cell pellet was resuspended in 4ml lysozyme medium (L-medium, Thompson et al., 1982, J. Bacteriol., Vol. 151, pp. 668-677) and incubated at 30°C for 30 minutes. A 5 ml aliquot of protoplast medium (P-medium, Okanishi, et al., 1974, J. Gen. Microbiol., Vil. 80, pp. 389-400) was added and the solution mixed by pipetting up and down. Protoplasts were filtered through glass wool (which traps mycelia but allows protoplasts to pass through) harvested by centrifugation, and resuspended in 2 ml P-medium. Protoplasts were used fresh or stored frozen in P-medium at -70°C.

Up to 3 µl of ligated plasmid DNA in ligation buffer was mixed gently with 50 µl S. lividans protoplasts in a small sterile tube. To this mixture, 200 µl transformation buffer (T-buffer, Thompson, et al., 1982, J. Bacteriol., Vol. 151, pp. 668-667) was added and pipetted to mix. Immediately, the cells were spread gently onto R2YE medium (Thompson, et al., 1980, Nature (London), Vol. 286, pp. 525-527) and incubated overnight at 30°C. The regenerated protoplasts were overlaid with filter paper (Whatman 541, 9.0cm) containing 100 µg/ml thiostrepton and incubated at 30°C for 3 days. About 200-500 transformants per plate were obtained using these transformation conditions. Approximately 12,000 thiostrepton-resistant S. lividans transformants were obtained in this way. Over 70% of these transformants contained recombinant pIJ702 plasmids as estimated by miniprep DNA analysis. These recombinant plasmids represented the genomic library of Rhodococcus 10554 DNA.

Screening for Cholesterol Oxidase Activit

As an example, showing the expression of one DNA sequence derived from a Nocardioform microorganism, recombinant S. lividans cells containing the genomic library of Rhodococcus 10554 DNA were screened for the expression of cholesterol oxidase activity. Cholesterol oxidase was determined using a modified method of Allain et al., 1974, Physical Chem., Vol. 20, pp. 470-475. Filter paper discs were scaked in an assay buffer containing 4-aminoantipyrine, phenol, horse radish peroxidase and triton®-X100, and laid down on colonies growing on agar medium containing cholesterol substrate. Alternatively, these colonies could be assayed by halo formation on agar medium containing cholesterol, in which halo formation depended on the conversion of cholesterol to cholestenone by soluble cholesterol oxidase. Recombinant S. lividans colonies which produced cholesterol oxidase were purified, and characterized.

Details of the screening of the genomic library for cholesterol oxidase activity were as follows: Filter papers used to apply thiostrepton drug to R2YE transformation plates for the selection of S. lividans transformants, were lifted and placed onto screening plates [medium containing per liter: agar, 15g; glycerol, 20g; yeast extract, 1g; $MgSO_4$, 0.66g; buffer salts, 66 ml (per liter: $Na_2HPO_4$, 28g; $NaH_2PO_4$, 30g; $K_2HPO_4$, 20g; $(NH_4)_2SO_4$, 127.5 g) and trace salts, 2 ml (per liter: concentrated HCl, 250 ml; $CaCl_2$, 3.57g; $ZnSO_4$, 20g; $CuCl_2$, 0.85g; $NaMoO_4$, 4.8g; $MnCl_2$, 2.0g; $FeCl_3$, 5.4g; boric acid, 0.3g; $CoCl_2$, 2.4g)] with cholesterol (1.6mM, Sigma) and thiostrepton (50 µg/ml). These replica plates were incubated at 30°C for 3 days. The replica filters were removed and replaced with sterile filter paper discs soaked in cholesterol oxidase assay reagent (100mM citric acid buffer, pH 6.0; 0.2mM 4-aminoantipyrine (Aldrich); horse radish peroxidase I, 600 units per liter; 6.4mM cholesterol; 10mM phenol and 4% w/v Triton® x 100). The plates were incubated at 30°C, and the colonies were screened over a 4 hour period for the development of a red zone due to cholesterol oxidase activity which diffused onto the white filter paper. After incubation at 30°C for 4 hours, the assay reagent filter papers were removed from the screening plates, and the plates were re-incubated at 30°C overnight to allow for the formation of clear halos associated with cholesterol oxidase degradation of cholesterol. From the approximately 8,000 recombinant S. lividans colonies screened, two showed cholesterol oxidase activity. The plasmid DNA from one of these isolates was called pSL81 and was analyzed further.

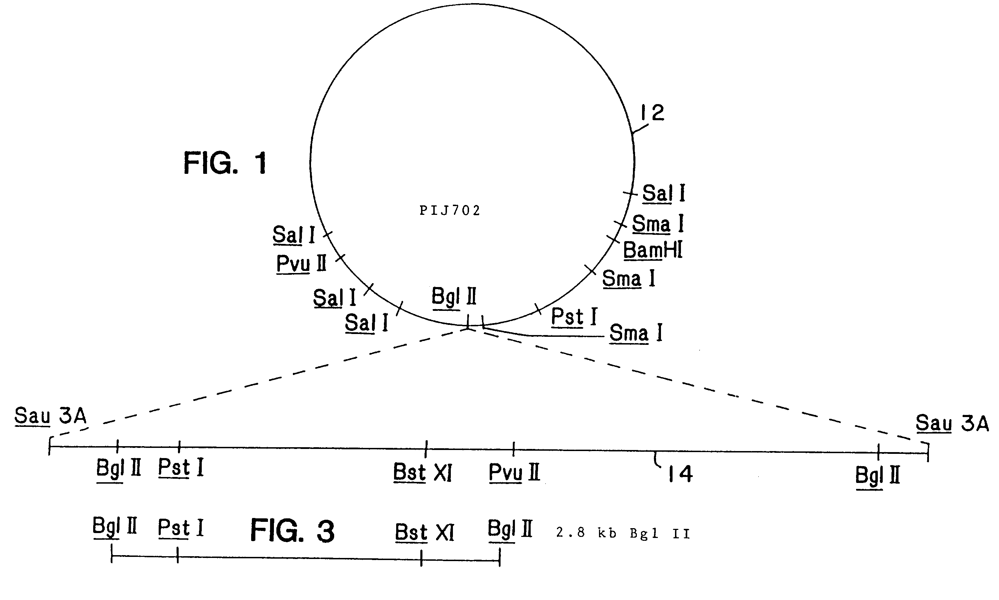

Plasmid DNA from the S. lividans/pSL81 recombinant was isolated and transformed back into S. lividans using the same procedure described for isolating and transforming pIJ702. All the thiostrepton-resistant transformants obtained contained the same plasmid pIJ702 with approximately 6 kb insert DNA, and showed the cholesterol oxidase activity diagnostic of the original recombinant. Curing of plasmid pSL81 from S. lividans cells by growth in the absence of drug selective pressure resulted in the simultaneous loss of thiostrepton resistance and cholesterol oxidase activity. Southern hybridization demonstrated that the insert DNA was derived from the Rhodococcus 10554 genome. Referring to Fig. 1, the recombinant plasmid designated pSL81 includes cloning vector pIJ702 12 and insert DNA 14 from Rhodococcus 10554, and has restriction endonuclease sites as indicated.

Plasmid pSL81, prepared as above, was used to probe chromosomal digests from the cholesterol oxidase producing Nocardioforms Rhodococcus NCIB 10554, R. erythropolis NCIB 9158, Nocardia erythropolis ATCC 17895 and Nocardia erythropolis ATCC 4277. The strains all showed cross-hybridizing DNA bands suggesting that the cholesterol oxidase coding region in these Nocardioforms is the same. Plasmid pSL81 showed no specific cross hybridization with DNA isolated from a cholesterol oxidase producing Streptomyces, namely Streptomyces violascene NRRL B-2700.

DNA Sequencing

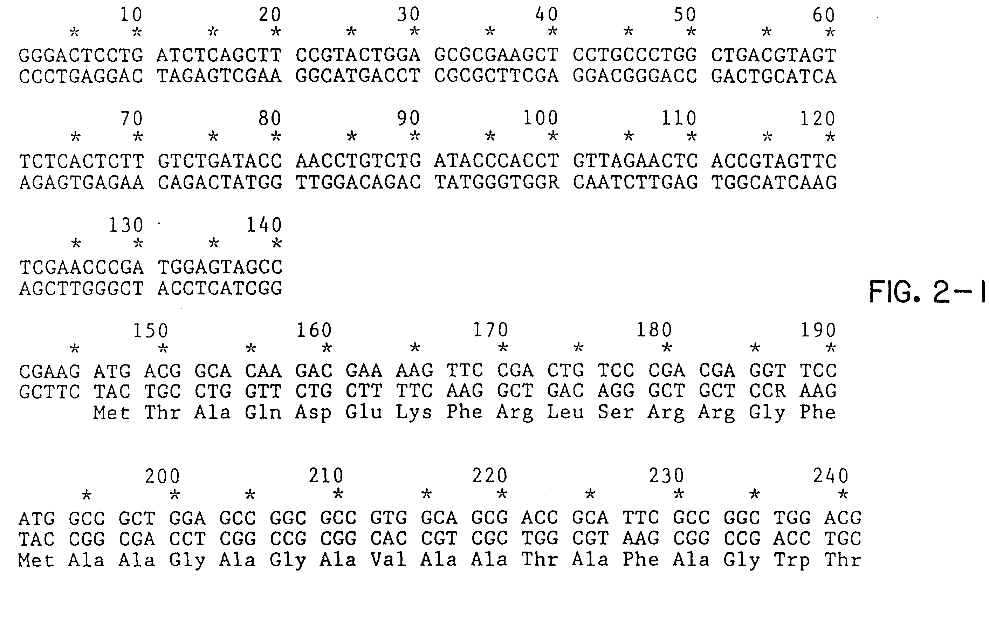

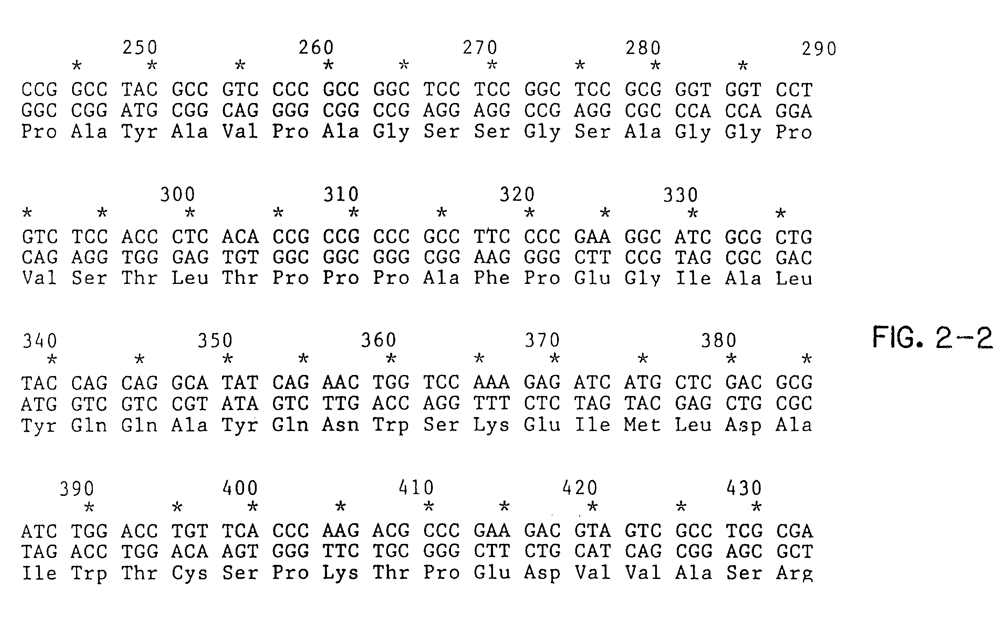

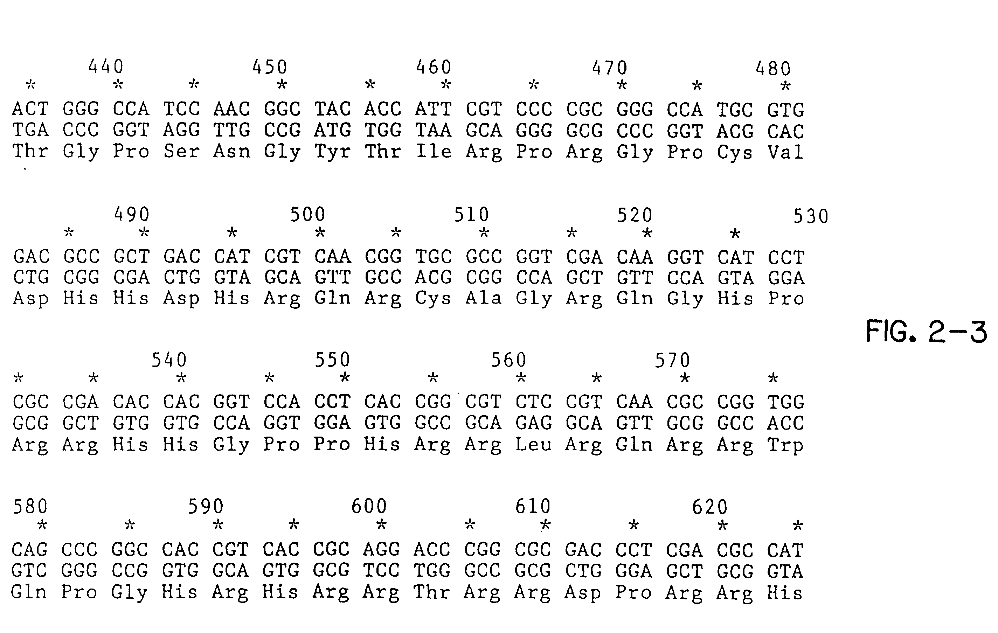

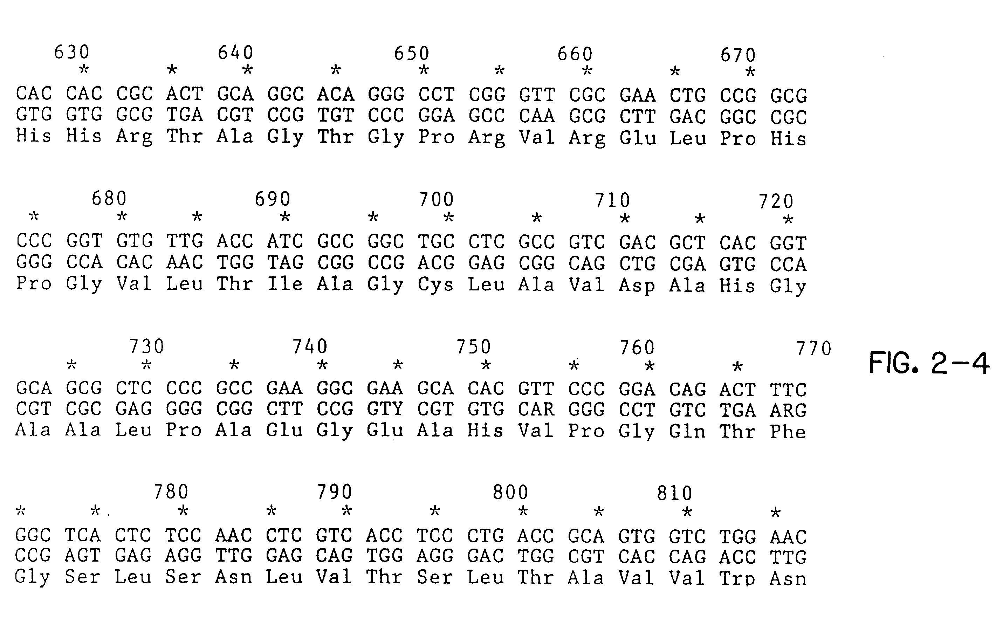

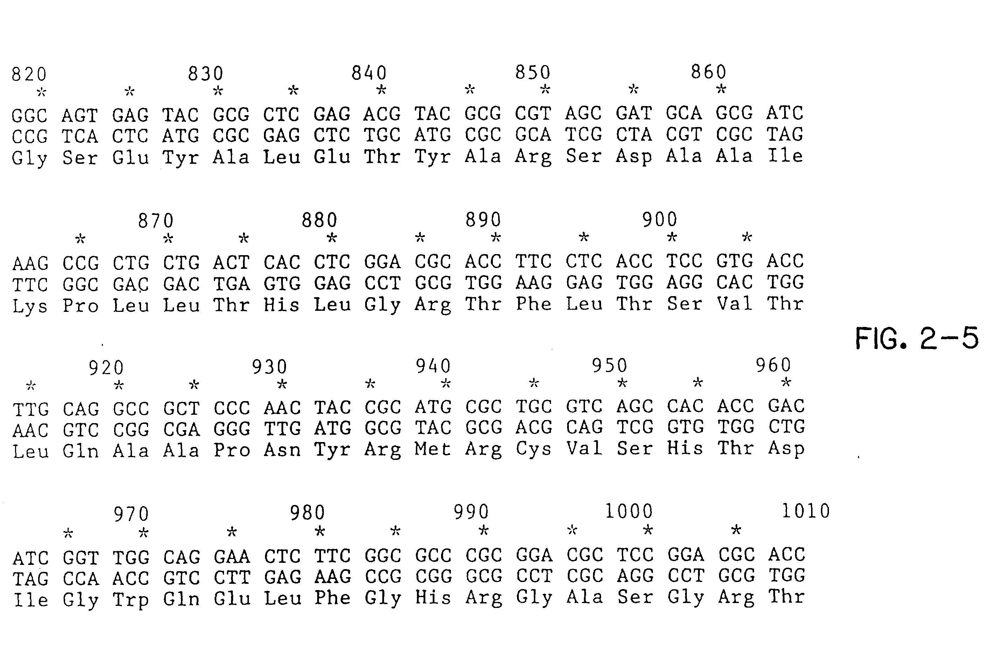

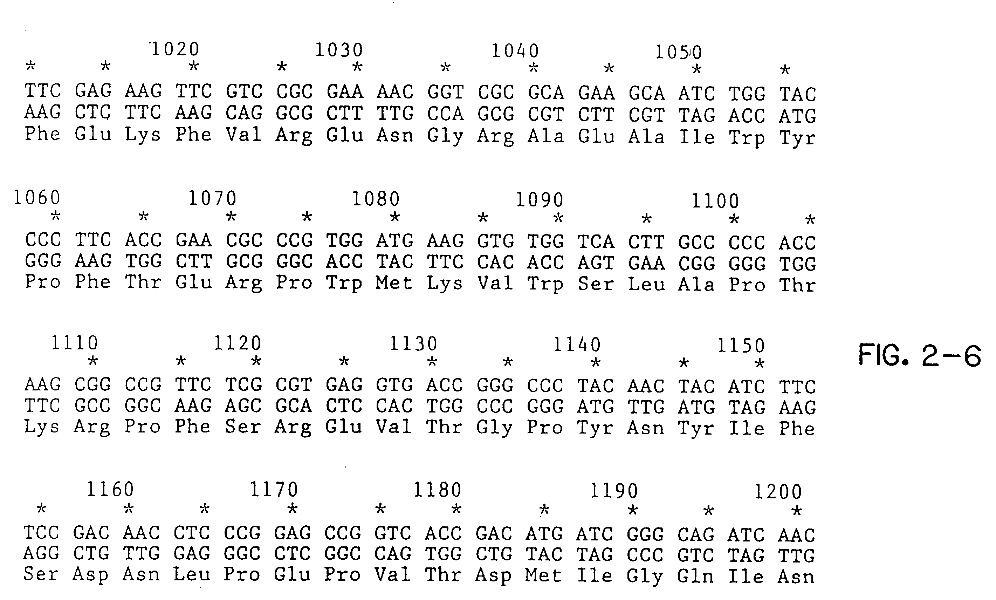

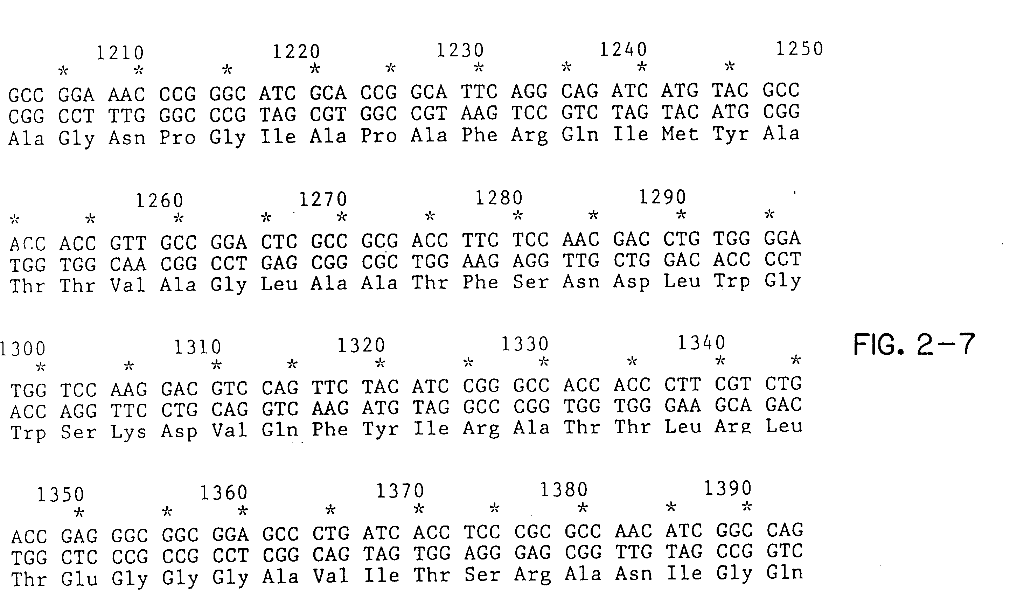

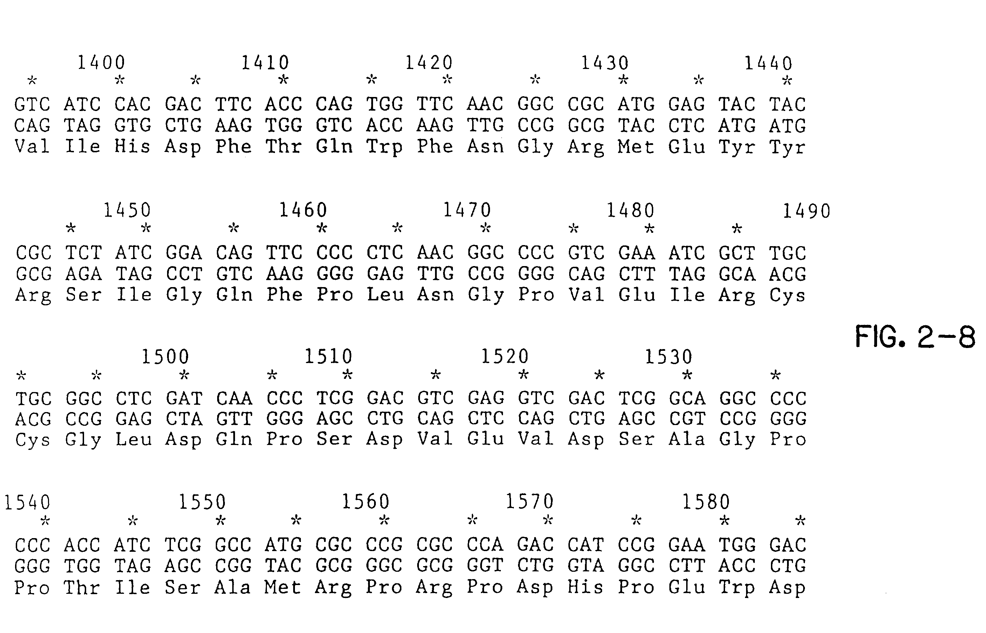

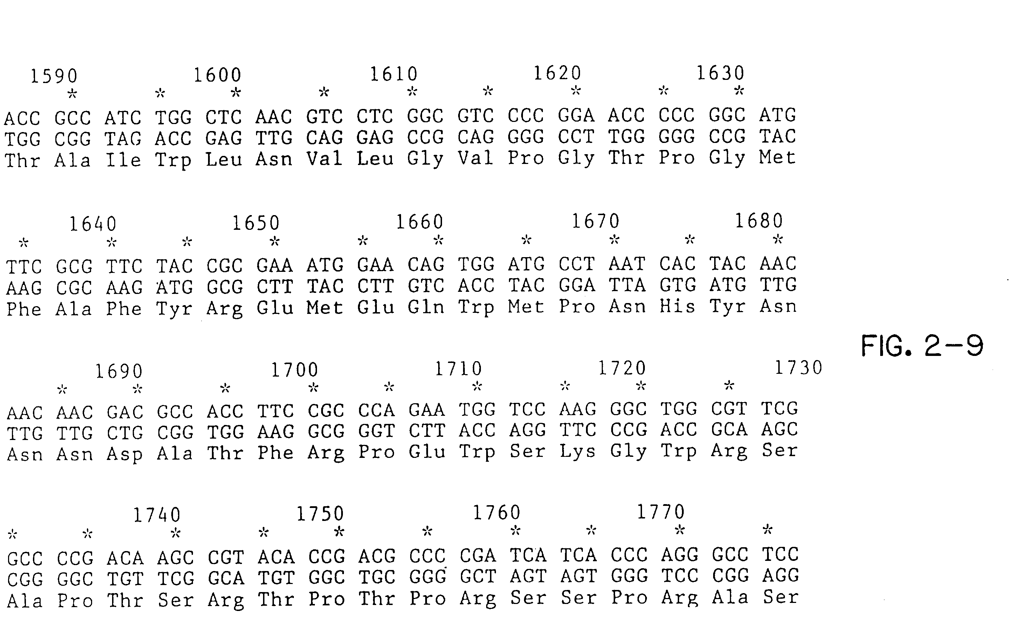

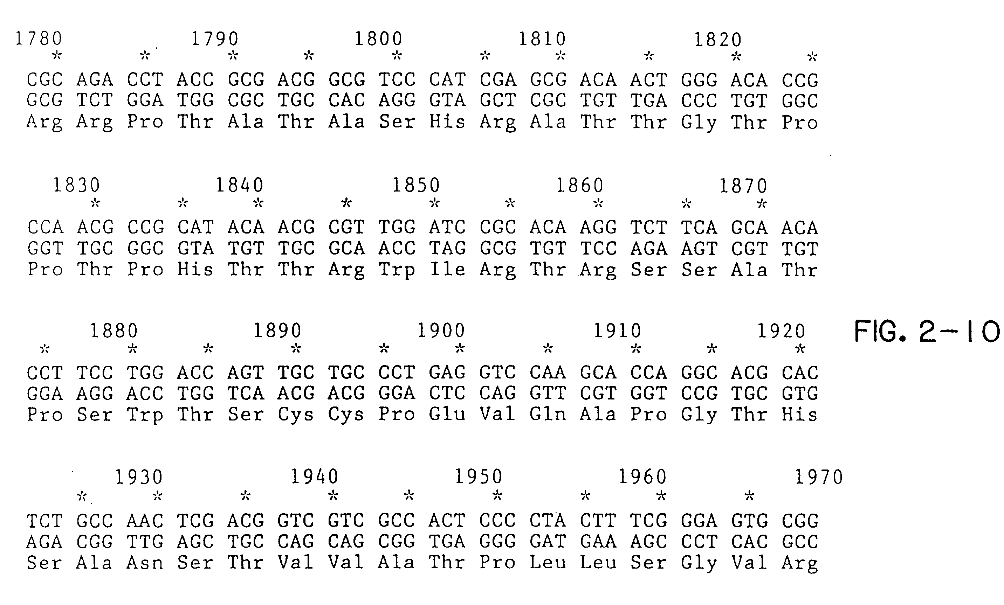

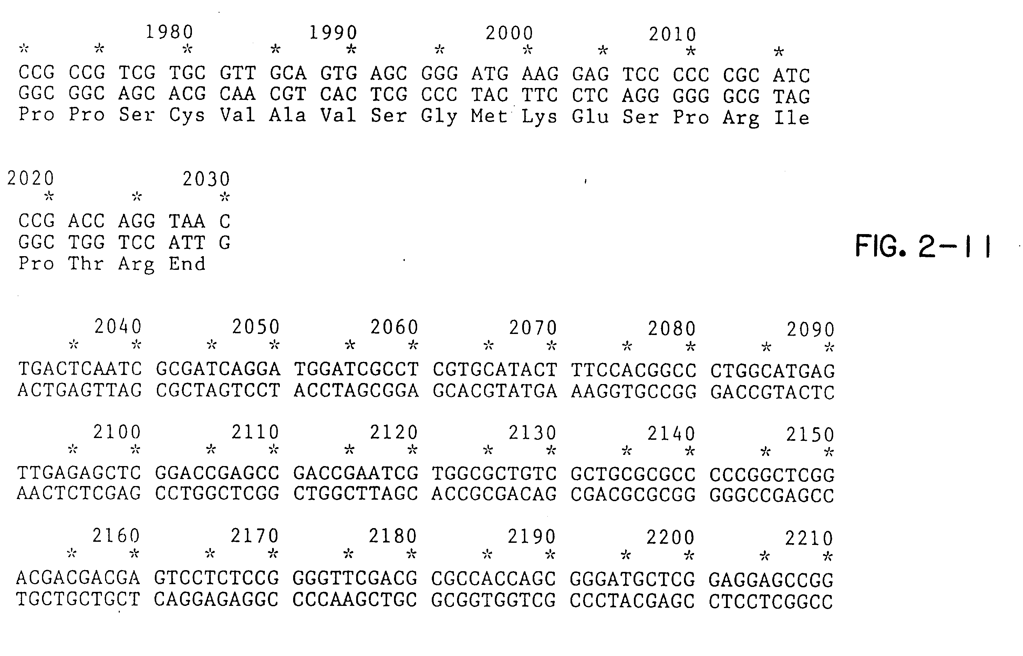

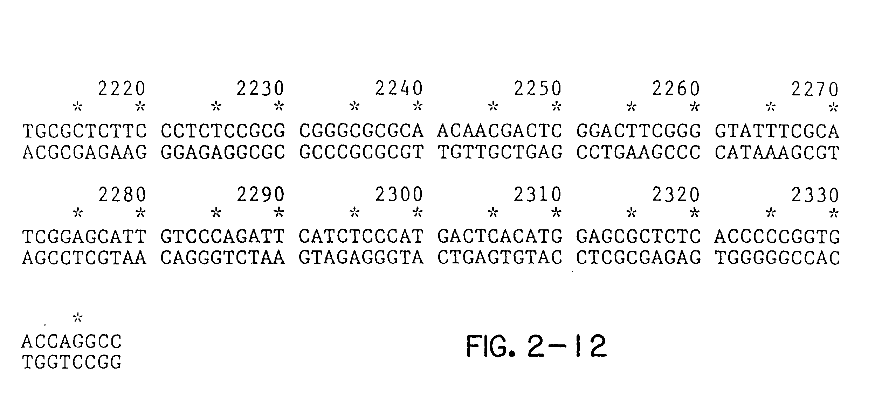

In order to sequence the cholesterol oxidase gene, subclones of pSL81 were constructed and a cholesterol oxidase coding region was localized to a 2.8 kb BglII fragment in the subclone pSL81∆15 (Fig. 3). This fragment was then introduced into E. coli on plasmid pBR322, and a series of deletion derivatives were constructed using available restriction sites. Sequencing was performed on denatured double stranded plasmid DNA using the dideoxy chain termination method of Sanger et al., PNAS USA, Vol. 74, pp. 5463-67. Specifically, a Sequenase Version 2.0 kit (United States Biochemical Corp.) was used according to the manufacturer's specifications. Deoxyguanidine triphosphate or Deoxy-7-deazoguanidine triphosphate was used to terminate chain polymerization reactions depending on the degree of G+C content of a particular region of DNA being sequenced. The primer extended oligonucleotides were electrophoresed on denaturing 6% polyacrylamide and the DNA sequences of adjacent DNA fragments were pieced together to construct a complete DNA sequence of the cholesterol oxidase gene. The sequence is shown in Fig. 2.

Subcloning of the Rhodococcus cholesterol oxidase gene in pSL81∆15 can result in an increase in expression of the cholesterol oxidase protein. Recombinant S. lividans cells were grown on RZYE agar containing 50µg/ml thiostrepton. After 2-3 days incubation at 30°C, the mycelia and spores were scraped from the surface of the agar and used to inoculate 50 ml YEME + 12.5 µg/ml thiostrepton seed cultures. These cultures were grown at 30°C with vigorous shaking and after 48 h, 2 l fermenters containing YEME + 12.5 µg/ml thiostrepton were inoculated with the seed. The fermentations were controlled at 28°C and pH 7.0 with aeration at 0.5 l/min and agitation at 1000 rpm. Cholesterol oxidase activity peaked after 48 h and in some such cultures expression was substantially greater in the S. lividans/pSL81∆15 fermentations than in the S. lividans/pSL81 fermentations.

Protein characterization

Recombinant cholesterol oxidase was isolated from 3 day old cultures of S. lividans/pSL81 cells grown in YEME (25 ml) containing thiostrepton (50 µg/ml). Non-recombinant cholesterol oxidase was isolated from 48 hour Rhodococcus 10554 cultures grown in liquid production medium which was the same medium as that used for the screening plates not solidified with agar. Induction experiments were performed using the same growth conditions in the presence of sterol as an inducer. Intracellular and extracellular fractions were recovered by centrifugation and assayed for cholesterol oxidase activity using a cholesterol oxidase specific diagnostic assay. The specificity of the recombinant cholesterol oxidase from S. lividans and the purified cholesterol oxidase from Rhodococcus 10554 were compared using various sterol substrates. Both enzymes showed similar profiles with the order of activity being cholesterol, pregnenolone, dihydroxycholesterol, stigmasterol and ergosterol.

Recombinant cholesterol oxidase from S. lividans/pSL81 was functional in standard diagnostic assays for the determination of cholesterol. An aliquot of ceil-free supernatant from a 3 day old culture of YEME grown recombinant cells was mixed with the cholesterol oxidase assay reagent (1.4 ml of 0.33 mg/ml 4-aminophenazone in 0.1 M phosphate buffer, pH 7.0, 0.05% Triton® X-100 (w/v); 1.4 ml of 1 mg/ml phenol in phosphate-Triton® X buffer; 0.1 ml of 4mM cholesterol; and 0.01 ml of 10 mg/ml peroxidase) and incubated at 30°C. Hydrogen peroxide formed during the oxidation of cholesterol and chelated with the aminoantipyrine in the assay mixture was determined by measuring the absorption of the red complex at 505nm versus a reagent blank containing water. The rate of cholesterol degradation by the recombinant enzyme was also measured directly by following the increase in absorbance at 240 nm owing to $\Delta^4$-cholestenone production. To 2.9 ml of sodium phosphate buffer (0.1M, pH 7.0) in a cuvette, 100 µl of 3% Triton X-100 (w/v) and 50 µl of a 6mM cholesterol solution were added. Diluted enzyme at approximately 0.2 U/ml in buffer was added and the absorbance at 240nm monitored using a Beckman spectrophotometer. Cholesterol oxidase activity was calculated as:

$$\text{units/mg} = \frac{\text{OD/min x RV}}{12.3 \text{ x DF x SV x PC (mg/ml)}},$$

where

RV = reaction volume
DF = dilution factor
SV = sample volume
PC = protein concentration

Further characterization of the recombinant cholesterol oxidase from S. lividans/pSL81 demonstrated that the enzyme could also be used to determine cholesterol in sera using the peroxidase coupled reaction.

Recombinant cholesterol oxidase from S. lividans/pSL81 and purified Rhodococcus 10554 cholesterol oxidase were analyzed by polyacrylamide gel electrophoresis using a 10% polyacrylamide gel and the buffer system of Laemelli, 1970, Nature, Vol. 227, pp. 680 et seq. Both enzymes were determined to be approximately 55 kD in size when compared with Biorad molecular weight standards.

Peptide sequencing

Recombinant cholesterol oxidase from S. lividans/pSL81Δ15 and non-recombinant cholesterol oxidase from R. rhodococcus were isolated as single bands from a polyacrylamide gel, and the amino-terminal peptide sequences were determined using a protein sequenator. Two peptide sequences were recovered from the recombinant protein band:

$$\text{NH}_2\text{-Gly-Gly-Pro-Val-Ser-Thr-Leu-Thr-Pro-Pro-Pro-Ala-Phe-,}$$

and

$$\text{NH}_2\text{-Gly-Pro-Val-Ser-Thr-Leu-Thr-Pro-Pro-Pro-Ala-Phe-.}$$

These peptide sequences are identical to those deduced directly from the DNA sequence between positions 280 and 320, and between positions 283 and 320, respectively. The elimination of a glycine residue at the N-terminus of one recombinant sequence is probably owing to a difference in post-translational processing by the host cell.

Amino terminal sequencing of cholesterol oxidase from R. rhodococcus identified the sequence

```
NH₂-Thr-Pro-Pro-Pro-Ala-Phe-Pro-Glu-Gly-Ile-Ala-Leu-Tyr-
Gln-Gln-,
```

which corresponds to the peptide sequence deduced from the DNA sequence between positions 300 and 346. Therefore, the enzyme found in the S. lividans/pSL81Δ15 culture medium is either 6 or 7 amino acids longer that the membrane bound enzyme found in R. rhodococcus.

Processing of gram-positive secreted proteins often involves signal sequence cleavage followed by hydrolysis at a secondary processing site. In the case of native cholesterol oxidase this secondary site is at the threonine. Apparently such a secondary cleavage does not occur when the protein is synthesized and secreted extracellularly by S. lividans.

Other Embodiments

Other embodiments are within the following claims. For example, other species of any genus of the Nocardioforms including Rhodococcus NCIB 10554, R. erythropolis NCIB 9158, Nocardia erythropolis ATCC 17895 and Nocardia erythropolis ATCC 4277 could be used to prepare the genomic library; or the libraries could be screened for other useful metabolic activities such as the assimilation of alkanes and aromatic hydrocarbons; the degradation of lignin, detergents, and pesticides; the production of enzymes useful in xenobiotic transformation; the biosynthesis of antibiotics, amino acids and biosurfactants; and the production of other steroid modifying enzymes such as cholesterol esterase.

As other Streptomyces and also Bacillus species share the advantages of S. lividans for the expression of heterologous genes, they could also serve as suitable hosts for the expression of DNA sequences derived from Nocardioform microorganisms. Other bacteria belonging to the high G+C subdivision of the gram-positive bacteria including any of the Nocardioforms, Mycobacterium, Corynebacterium, and Athrobacter would be suitable hosts for cloning DNA sequences derived from Nocardioform microorganisms.

S. lividans/pSL81 can undergo rearrangement in one or more regions outside the cholesterol oxidase coding region and can retain cholesterol oxidase transforming activity. One such S. lividans/pSL81 rearranged plasmid, has at least 3.6 kb of the 6 kb Rhodococcus DNA unchanged as determined by restriction mapping. In this rearranged S. lividans/pSL81 plasmid a DNA insertion of approximately 2.5 kb has occurred in the region to the right of the PvuII site. Cholesterol oxidase was recoverable from cultures of S. lividans transformed with this rearranged plasmid, in lower quantities than from S. lividans/pSL81 cultures.

In still other embodiments expression of cholesterol oxidase may be increased by further manipulating the pSL81Δ15 clone, as will be apparent to one skilled in the art. For example, a higher copy number vector could be used, such as, for example, pIJ303, described in Hopwood et al., 1985, Genetic Manipulation of Streptomyces: A Laboratory Manual. Gene expression could also be enhanced by replacing the cholesterol oxidase promoter with a stronger exogenous promoters, such as ErmE promoter, described in Bibb et al., 1985, Gene, Vol. 38, pp. 215-226; aph promoter, described in Hopwood et al., 1985; or a tac promoter, described in Amman et al., 1984, Gene, Vol. 25, pp. 167 et seq.

Deposits

Under the terms of the Budapest Treaty on the International Recognition of the Deposit of Microorganisms for the Purpose of Patent Procedure, deposit of plasmid pSL81 has been made with the American Type Culture Collection (ATCC) of Rockville, MD, USA.

**Claims**

1. A cloning vector comprising a DNA sequence derived from a Nocardioform microorganism, said DNA sequence encoding a cholesterol oxidase, and said cloning vector being capable of expressing said DNA sequence in a host Streptomyces microorganism.

2. The cloning vector of Claim 1 wherein said Nocardioform microorganism is Rhodococcus sp. NCIB 10554.

3. The cloning vector of Claim 1 wherein said host microorganism is S. lividans.

4. The cloning vector of Claim 1, wherein said DNA sequence encodes an amino acid sequence comprising the sequence:

Thr Pro Pro Pro Ala Phe Pro Glu Gly Ile Ala Leu Tyr Gln Gln.

**5.** The cloning vector of Claim 1, wherein said DNA sequence encodes an amino acid sequence comprising the sequence:

Gly Pro Val Ser Thr Leu Thr Pro Pro Pro Ala Phe Pro Glu Gly Ile Ala Leu Tyr Gln Gln.

**6.** The cloning vector of Claim 1, wherein said DNA sequence encodes an amino acid sequence comprising the sequence:

Gly Gly Pro Val Ser Thr Leu Thr Pro Pro Pro Ala Phe Pro Glu Gly Ile Ala Leu Tyr Gln Gln.

**7.** The cloning vector of Claim 1, wherein said DNA sequence encodes an amino acid sequence comprising the sequence:

Met Thr Ala Gln Asp Glu Lys Phe Arg Leu Ser Arg
Arg Gly Phe Met Ala Ala Gly Ala Gly Ala Val Ala Ala Thr
Ala Phe Ala Gly Trp Thr Pro Ala Tyr Ala Val Pro Ala Gly
Ser Ser Gly Ser Ala Gly Gly Pro Val Ser Thr Leu Thr Pro
Pro Pro Ala Phe Pro Glu Gly Ile Ala Leu Tyr Gln Gln Ala
Tyr Gln Asn Trp Ser Lys Glu Ile Met Leu Asp.

**8.** The cloning vector of Claim 1, wherein said DNA sequence comprises the sequence:

ACA CCG CCG CCC GCC TTC CCC GAA GGC ATC GCG CTG TAC CAG CAG,

which encodes the amino acid sequence referred to in Claim 4.

**9.** The cloning vector of Claim 1, wherein said DNA sequence comprises the sequence:

GGT CCT GTC TCC ACC CTC ACA CCG CCG CCC GCC TTC CCC GAA GGC ATC GCG CTG TAC CAG CAG,

referred to in Claim 5.

**10.** The cloning vector of Claim 1, wherein said DNA sequence comprises the sequence:

GGT GGT CCT GTC TCC ACC CTC ACA CCG CCG CCC GCC TTC CCC GAA GGC ATC GCG CTG TAC CAG CAG,

sequence referred to in Claim 6.

**11.** The cloning vector of Claim 1, wherein said DNA sequence comprises the sequence:

```
CGAAG ATG ACG GCA CAA GAC GAA AAG TTC CGA CTG TCC CGA
CGA GGT TTC ATG GCC GCT GGA GCC GGC GCC GTG GCA GCG ACC
GCA TTC GCC GGC TGG ACG CCG GCC TAC GCC GTC CCC GCC GGC
TCC TCC GGC TCC GCG GGT GGT CCT GTC TCC ACC CTC ACA CCG
CCG CCC GCC TTC CCC GAA GGC ATC GCG CTG TAC CAG CAG GCA
TAT CAG AAC TGG TCC AAA GAG ATC ATG CTC GAC
```

**12.** The cloning vector of Claim 1, wherein said DNA sequence comprises the sequence:

```
GGGACTCCTG ATCTCAGCTT CCGTACTGGA GCGCGAAGCT CCTGCCCTGG
CTGACGTAGT TCTCACTCTT GTCTGATACC AACCTGTCTG ATACCCACCT
GTTAGAACTC ACCGTAGTTC TCGAACCCGA TGGAGTAGCC CGAAG ATG
ACG GCA CAA GAC GAA AAG TTC CGA CTG TCC CGA CGA GGT TTC
ATG GCC GCT GGA GCC GGC GCC GTG GCA GCG ACC GCA TTC GCC
GGC TGG ACG CCG GCC TAC GCC GTC CCC GCC GGC TCC TCC GGC
TCC GCG GGT GGT CCT GTC TCC ACC CTC ACA CCG CCG CCC GCC
TTC CCC GAA GGC ATC GCG CTG TAC CAG CAG GCA TAT CAG AAC
TGG TCC AAA GAG ATC ATG CTC GAC
```

**13.** The cloning vector of Claim 1, wherein said Nocardioform microorganism is R. erythropolis NCIB 9158.

**14.** The cloning vector of Claim 1, wherein said Nocardioform microorganism is Nocardia erythropolis ATCC 17895.

**15.** The cloning vector of Claim 1, wherein said Nocardioform microorganism is Nocardia erythropolis ATCC 4277.

**16.** The vector of Claim 1, further comprising an ErmE promoter.

**17.** The vector of Claim 1, further comprising an aph promoter.

**18.** The vector of Claim 1, further comprising a tac promoter.

**19.** The plasmid pSL81, as featured in Figure 2.

**20.** A cell of a species of Streptomyces transformed with the cloning vector of Claim 1.

**21.** A cell of the species S. lividans transformed with the cloning vector of Claim 3.

**22.** A method for producing cholesterol oxidase comprising

providing a cloning vector containing a DNA sequence derived from a Nocardioform microorganism, said DNA sequence encoding cholesterol oxidase,
transforming a Streptomyces host cell with said cloning vector to obtain a recombinant host cell,
culturing said recombinant host cell under conditions permitting expression of said DNA sequence, and recovering the cholesterol oxidase.

**23.** The method of Claim 22, wherein said Nocardioform organism is Rhodococcus sp. NCIB 10554.

EP 0 426 768 B1

**24.** The method of Claim 23, wherein said host cell is <u>S. lividans</u>.

**25.** The method of Claim 23, wherein said cloning vector is plasmid pSL81, as featured in Figure 2.

**Patentansprüche**

**1.** Klonierungsvektor, umfassend eine DNA-Sequenz, die sich von einem Nocardioform-Mikroorganismus ableitet, wobei die DNA-Sequenz eine Cholesterinoxidase codiert und der Klonierungsvektor die DNA-Sequenz in einem <u>Streptomyces</u>-Mikroorganismus als Wirt exprimiert.

**2.** Klonierungsvektor nach Anspruch 1, dadurch **gekennzeichnet**, daß der Nocardioform-Mikroorganismus <u>Rhodococcus</u> <u>sp</u>. NCIB 10554 ist.

**3.** Klonierungsvektor nach Anspruch 1, dadurch **gekennzeichnet**, daß der Wirtmikroorganismus S. <u>lividans</u> ist.

**4.** Klonierungsvektor nach Anspruch 1, dadurch **gekenzeichnet**, daß die DNA-Sequenz eine Aminosäuresequenz codiert, die die Sequenz Thr Pro Pro Pro Ala Phe Pro Glu Gly Ile Ala Leu Tyr Gln Gln umfaßt.

**5.** Klonierungsvektor nach Anspruch 1, dadurch **gekennzeichnet**, daß die DNA-Sequenz eine Aminosäuresequenz codiert, die die Sequenz Gly Pro Val Ser Thr Leu Thr Pro Pro Pro Ala Phe Pro Glu Gly Ile Ala Leu Tyr Gln Gln umfaßt.

**6.** Klonierungsvektor nach Anspruch 1, dadurch **gekennzeichnet**, daß die DNA-Sequenz eine Aminosäuresequenz codiert, die die Sequenz Gly Gly Pro Val Ser Thr Leu Thr Pro Pro Pro Ala Phe Pro Glu Gly Ile Ala Leu Tyr Gln Gln umfaßt.

**7.** Klonierungsvektor nach Anspruch 1, dadurch **gekennzeichnet**, daß die DNA-Sequenz eine Aminosäuresequenz codiert, die die Sequenz

```
      Met Thr Ala Gln Asp Glu Lys Phe Arg Leu Ser Arg
   Arg Gly Phe Met Ala Ala Gly Ala Gly Ala Val Ala Ala Thr
   Ala Phe Ala Gly Trp Thr Pro Ala Tyr Ala Val Pro Ala Gly
   Ser Ser Gly Ser Ala Gly Gly Pro Val Ser Thr Leu Thr Pro
   Pro Pro Ala Phe Pro Glu Gly Ile Ala Leu Tyr Gln Gln Ala
   Tyr Gln Asn Trp Ser Lys Glu Ile Met Leu Asp.
```

umfaßt.

**8.** Klonierungsvektor nach Anspruch 1, dadurch **gekennzeichnet**, daß die DNA-Sequenz die Sequenz ACA CCG CCG CCC GCC TTC CCC GAA GGC ATC GCG CTG TAC CAG CAG umfaßt, welche die Aminosäuresequenz in Anspruch 4 codiert.

**9.** Klonierungsvektor nach Anspruch 1, dadurch **gekennzeichnet**, daß die DNA-Sequenz die Sequenz GGT CCT GTC TCC ACC CTC ACA CCG CCG CCC GCC TTC CCC GAA GGC ATC GCG CTG TAC CAG CAG umfaßt, die die Aminosäuresequenz nach Anspruch 5 codiert.

**10.** Klonierungsvektor nach Anspruch 1, dadurch **gekennzeichnet**, daß die DNA-Sequenz die Sequenz GGT GGT CCT GTC TCC ACC CTC ACA CCG CCG CCC GCC TTC CCC GAA GGC ATC GCG CTG TAC CAG CAG umfaßt, die die Aminosäuresequenz von Anspruch 6 codiert.

**11.** Klonierungsvektor nach Anspruch 1, dadurch **gekennzeichnet**, daß die DNA-Sequenz die Sequenz:

10

```
CGAAG ATG ACG GCA CAA GAC GAA AAG TTC CGA CTG TCC CGA
CGA GGT TTC ATG GCC GCT GGA GCC GGC GCC GTG GCA GCG ACC
GCA TTC GCC GGC TGG ACG CCG GCC TAC GCC GTC CCC GCC GGC
TCC TCC GGC TCC GCG GGT GGT CCT GTC TCC ACC CTC ACA CCG
CCG CCC GCC TTC CCC GAA GGC ATC GCG CTG TAC CAG CAG GCA
TAT CAG AAC TGG TCC AAA GAG ATC ATG CTC GAC
```

umfaßt.

**12.** Klonierungsvektor nach Anspruch 1, dadurch **gekenzeichnet**, daß die DNA-Sequenz die Sequenz

```
GGGACTCCTG ATCTCAGCTT CCGTACTGGA GCGCGAAGCT CCTGCCCTGG
CTGACGTAGT TCTCACTCTT GTCTGATACC AACCTGTCTG ATACCCACCT
GTTAGAACTC ACCGTAGTTC TCGAACCCGA TGGAGTAGCC CGAAG ATG
ACG GCA CAA GAC GAA AAG TTC CGA CTG TCC CGA CGA GGT TTC
ATG GCC GCT GGA GCC GGC GCC GTG GCA GCG ACC GCA TTC GCC
GGC TGG ACG CCG GCC TAC GCC GTC CCC GCC GGC TCC TCC GGC
TCC GCG GGT GGT CCT GTC TCC ACC CTC ACA CCG CCG CCC GCC
TTC CCC GAA GGC ATC GCG CTG TAC CAG CAG GCA TAT CAG AAC
TGG TCC AAA GAG ATC ATG CTC GAC
```

umfaßt.

**13.** Klonierungsvektor nach Anspruch 1, dadurch **gekennzeichnet**, daß der Nocardioform-Mikroorganismus R. erythropolis NCIB 9158 ist.

**14.** Klonierungsvektor nach Anspruch 1, dadurch **gekennzeichnet**, daß der Nocardioform-Mikroorganismus Nocardia erythropolis ATCC 17895 ist.

**15.** Klonierungsvektor nach Anspruch 1, dadurch **gekennzeichnet**, daß der Nocardioform-Mikroorganismus Nocardia erythropolis ATCC 4277 ist.

**16.** Vektor nach Anspruch 1, dadurch **gekennzeichnet**, daß er weiter einen ErmE-Promotor umfaßt.

**17.** Vektor nach Anspruch 1, dadurch **gekennzeichnet**, daß er weiter einen aph-Promotor umfaßt.

**18.** Vektor nach Anspruch 1, dadurch **gekennzeichnet**, daß er weiter einen tac-Promotor umfaßt.

**19.** Plasmid pSL81, wie in Figur 2 dargestellt.

**20.** Zelle eines Spezies von Streptomyces, transformiert mit dem Klonierungsvektor von Anspruch 1.

**21.** Zelle des Spezies S. lividans, transformiert mit dem Klonierungsvektor von Anspruch 3.

**22.** Verfahren zur Herstellung von Cholesterinoxidase, umfassend Bereitstellung eines Klonierungsvektors, enthaltend eine DNA-Sequenz, die sich von einem Nocardioform-Mikroorganismus ableitet, wobei die DNA-Sequenz Cholesterinoxidase codiert,

Transformierung einer <u>Streptomyces</u>-Wirtzelle mit dem Klonierungsvektor unter Bildung einer rekombinanten Wirtzelle,
Züchten der rekombinanten Wirtzelle bei Bedingungen, die die Exprimierung der DNA-Sequenz erlauben, und
Gewinnung der Cholesterinoxidase.

23. Verfahren nach Anspruch 22, dadurch **gekennzeichnet**, daß der Nocardioform-Organismus <u>Rhodococcus</u> sp. NCIB 10554 ist.

24. Verfahren nach Anspruch 23, dadurch **gekennzeichnet**, daß die Wirtzelle <u>S</u>. <u>lividans</u> ist.

25. Verfahren nach Anspruch 23, dadurch **gekennzeichnet**, daß der Klonierungsvektor das Plasmid pSL81, wie in Figur 2 dargestellt, ist.

**Revendications**

1. Vecteur de clonage comprenant une séquence d'ADN dérivée d'un microorganisme du groupe des Nocardioformes, ladite séquence d'ADN codant pour une cholestérol oxydase et ledit vecteur de clonage étant capable d'exprimer ladite séquence d'ADN dans un microorganisme hôte du genre <u>Streptomyces.</u>

2. Vecteur de clonage selon la revendication 1, dans lequel ledit microorganisme du groupe des Nocardioformes est <u>Rhodococcus</u> <u>sp</u>. NCIB 10554.

3. Vecteur de clonage selon la revendication 1, dans lequel ledit microorganisme hôte est <u>S.lividans.</u>

4. Vecteur de clonage selon la revendication 1, dans lequel ladite séquence d'ADN code pour une séquence d'acides aminés comprenant la séquence :

Thr Pro Pro Pro Ala Phe Pro Glu Gly Ile Ala Leu Tyr Gln Gln.

5. Vecteur de clonage selon la revendication 1, dans lequel ladite séquence d'ADN code pour une séquence d'acides aminés comprenant la séquence :

Gly Pro Val Ser Thr Leu Thr Pro Pro Pro Ala Phe Pro Glu Gly
Ile Ala Leu Tyr Gln Gln.

6. Vecteur de clonage selon la revendication 1, dans lequel ladite séquence d'ADN code pour une séquence d'acides aminés comprenant la séquence :

Gly Gly Pro Val Ser Thr Leu Thr Pro Pro Pro Ala Phe Pro Glu
Gly Ile Ala Leu Tyr Gln Gln.

7. Vecteur de clonage selon la revendication 1, dans lequel ladite séquence d'ADN code pour une séquence d'acides aminés comprenant la séquence :

Met Thr Ala Gln Asp Glu Lys Phe Arg Leu Ser Arg
Arg Gly Phe Met Ala Ala Gly Ala Gly Ala Val Ala Ala Thr

EP 0 426 768 B1

Ala Phe Ala Gly Trp Thr Pro Ala Tyr Ala Val Pro Ala Gly
Ser Ser Gly Ser Ala Gly Gly Pro Val Ser Thr Leu Thr Pro
Pro Pro Ala Phe Pro Glu Gly Ile Ala Leu Tyr Gln Gln Ala
Tyr Gln Asn Trp Ser Lys Glu Ile Met Leu Asp.

8. Vecteur de clonage selon la revendication 1, dans lequel ladite séquence d'ADN comprend la séquence :

ACA CCG CCG CCC GCC TTC CCC GAA GGC ATC GCG CTG TAC CAG CAG

qui code pour la séquence d'acides aminés mentionnée à la revendication 4.

9. Vecteur de clonage selon la revendication 1, dans lequel ladite séquence d'ADN comprend la séquence :

GGT CCT GTC TCC ACC CTC ACA CCG CCG CCC GCC TTC CCC GAA GGC
ATC GCG CTG TAC CAG CAG

qui code pour la séquence d'acides aminés mentionnée à la 15 revendication 5.

10. Vecteur de clonage selon la revendication 1, dans lequel ladite séquence d'ADN comprend la séquence :

GGT GGT CCT GTC TCC ACC CTC ACA CCG CCG CCC GCC TTC CCC GAA
GGC ATC GCG CTG TAC CAG CAG

qui code pour la séquence d'acides aminés mentionnée à la revendication 6.

11. Vecteur de clonage selon la revendication 1, dans lequel ladite séquence d'ADN comprend la séquence :

CGAAG ATG ACG GCA CAA GAC GAA AAG TTC CGA CTG TCC CGA
CGA GGT TTC ATG GCC GCT GGA GCC GGC GCC GTG GCA GCG ACC
GCA TTC GCC GGC TGG ACG CCG GCC TAC GCC GTC CCC GCC GGC
TCC TCC GGC TCC GCG GGT GGT CCT GTC TCC ACC CTC ACA CCG
CCG CCC GCC TTC CCC GAA GGC ATC GCG CTG TAC CAG CAG GCA
TAT CAG AAC TGG TCC AAA GAG ATC ATG CTC GAC

12. Vecteur de clonage selon la revendication 1, dans lequel ladite séquence d'ADN comprend la séquence :

GGGACTCCTG ATCTCAGCTT CCGTACTGGA GCGCGAAGCT CCTGCCCTGG
CTGACGTAGT TCTCACTCTT GTCTGATACC AACCTGTCTG ATACCCACCT
GTTAGAACTC ACCGTAGTTC TCGAACCCGA TGGAGTAGCC CGAAG ATG
ACG GCA CAA GAC GAA AAG TTC CGA CTG TCC CGA CGA GGT TTC

13

ATG GCC GCT GGA GCC GGC GCC GTG GCA GCG ACC GCA TTC GCC

GGC TGG ACG CCG GCC TAC GCC GTC CCC GCC GGC TCC TCC GGC

TCC GCG GGT GGT CCT GTC TCC ACC CTC ACA CCG CCG CCC GCC TTC CCC GAA GGC ATC GCG CTG TAC CAG CAG GCA TAT CAG AAC

TGG TCC AAA GAG ATC ATG CTC GAC

**13.** Vecteur de clonage selon la revendication 1, dans lequel ledit microorganisme du groupe des Nocardioformes est R. erythropolis NCIB 9158.

**14.** Vecteur de clonage selon la revendication 1, dans lequel ledit microorganisme du groupe des Nocardioformes est Nocardia erythropolis ATCC 17895.

**15.** Vecteur de clonage selon la revendication 1, dans lequel ledit microorganisme du groupe des Nocardioformes est Nocardia erythropolis ATCC 4277.

**16.** Vecteur selon la revendication 1, comprenant en outre un promoteur ErmE.

**17.** Vecteur selon la revendication 1, comprenant en outre un promoteur aph.

**18.** Vecteur selon la revendication 1, comprenant en outre un promoteur tac.

**19.** Plasmide pSL81 tel que caractérisé à la Figure 2.

**20.** Cellule du genre Streptomyces transformée avec le vecteur de clonage selon la revendication 1.

**21.** Cellule de l'espèce S. lividans transformée avec le vecteur de clonage selon la revendication 3.

**22.** Procédé de production de cholestérol oxydase comprenant les étapes consistant à :

procurer un vecteur de clonage contenant une séquence d'ADN dérivée d'un microorganisme du groupe des Nocardioformes, ladite séquence d'ADN codant pour la cholestérol oxydase,
transformer une cellule hôte du genre Streptomyces avec ledit vecteur de clonage pour obtenir une cellule hôte recombinante,
cultiver ladite cellule hôte recombinante dans des conditions permettant l'expression de ladite séquence d'ADN et récupérer la cholestérol oxydase.

**23.** Procédé selon la revendication 22, dans lequel ledit organisme du groupe des Nocardioformes est Rhodococcus sp. NCIB 10554.

**24.** Procédé selon la revendication 23, dans lequel la cellule hôte est S.lividans.

**25.** Procédé selon la revendication 23, dans lequel ledit vecteur de clonage est le plasmide pSL81 tel que caractérisé à la Figure 2.

EP 0 426 768 B1

FIG. 1

PIJ702

12

Sal I

Sma I

BamHI

Sma I

Sal I
Pvu II

Sal I
Sal I

Bgl II

Pst I
Sma I

Sau 3A

Sau 3A

Bgl II   Pst I                Bst XI   Pvu II              14              Bgl II

Bgl II   Pst I   FIG. 3       Bst XI   Bgl II   2.8 kb Bgl II

EP 0 426 768 B1

```
            10          20          30          40          50          60
       *      *      *      *      *      *      *      *      *      *      *      *
   GGGACTCCTG ATCTCAGCTT CCGTACTGGA GCGCGAAGCT CCTGCCCTGG CTGACGTAGT
   CCCTGAGGAC TAGAGTCGAA GGCATGACCT CGCGCTTCGA GGACGGGACC GACTGCATCA


            70          80          90         100         110         120
       *      *      *      *      *      *      *      *      *      *      *      *
   TCTCACTCTT GTCTGATACC AACCTGTCTG ATACCCACCT GTTAGAACTC ACCGTAGTTC
   AGAGTGAGAA CAGACTATGG TTGGACAGAC TATGGGTGGR CAATCTTGAG TGGCATCAAG


           130  ·      140
       *      *      *      *
   TCGAACCCGA TGGAGTAGCC
   AGCTTGGGCT ACCTCATCGG
```

FIG. 2-1

```
            150         160         170         180         190
       *      *      *      *      *      *      *      *      *      *
   CGAAG ATG ACG GCA CAA GAC GAA AAG TTC CGA CTG TCC CGA CGA GGT TCC
   GCTTC TAC TGC CTG GTT CTG CTT TTC AAG GCT GAC AGG GCT GCT CCR AAG
         Met Thr Ala Gln Asp Glu Lys Phe Arg Leu Ser Arg Arg Gly Phe


            200         210         220         230         240
       *      *      *      *      *      *      *      *      *      *
   ATG GCC GCT GGA GCC GGC GCC GTG GCA GCG ACC GCA TTC GCC GGC TGG ACG
   TAC CGG CGA CCT CGG CCG CGG CAC CGT CGC TGG CGT AAG CGG CCG ACC TGC
   Met Ala Ala Gly Ala Gly Ala Val Ala Ala Thr Ala Phe Ala Gly Trp Thr
```

EP 0 426 768 B1

FIG. 2-2

```
        250              260              270              280              290
 *       *        *       *        *       *        *       *        *
CCG GCC TAC GCC GTC CCC GCC GGC TCC TCC GGC TCC GCG GGT GGT CCT
GGC CGG ATG CGG CAG GGG CGG CCG AGG AGG CCG AGG CGC CCA CCA GGA
Pro Ala Tyr Ala Val Pro Ala Gly Ser Ser Gly Ser Ala Gly Gly Pro


        300              310              320              330
 *       *        *       *        *       *        *       *        *
GTC TCC ACC CTC ACA CCG CCG CCC GCC TTC CCC GAA GGC ATC GCG CTG
CAG AGG TGG GAG TGT GGC GGC GGG CGG AAG GGG CTT CCG TAG CGC GAC
Val Ser Thr Leu Thr Pro Pro Pro Ala Phe Pro Glu Gly Ile Ala Leu


340              350              360              370              380
 *       *        *       *        *       *        *       *        *       *
TAC CAG CAG GCA TAT CAG AAC TGG TCC AAA GAG ATC ATG CTC GAC GCG
ATG GTC GTC CGT ATA GTC TTG ACC AGG TTT CTC TAG TAC GAG CTG CGC
Tyr Gln Gln Ala Tyr Gln Asn Trp Ser Lys Glu Ile Met Leu Asp Ala


        390              400              410              420              430
 *       *        *       *        *       *        *       *        *
ATC TGG ACC TGT TCA CCC AAG ACG CCC GAA GAC GTA GTC GCC TCG CGA
TAG ACC TGG ACA AGT GGG TTC TGC GGG CTT CTG CAT CAG CGG AGC GCT
Ile Trp Thr Cys Ser Pro Lys Thr Pro Glu Asp Val Val Ala Ser Arg
```

```
          440              450              460              470              480
 *         *         *         *         *         *         *         *         *         *
ACT GGG CCA TCC AAC GGC TAC ACC ATT CGT CCC CGC GGG CCA TGC GTG
TGA CCC GGT AGG TTG CCG ATG TGG TAA GCA GGG GCG CCC GGT ACG CAC
Thr Gly Pro Ser Asn Gly Tyr Thr Ile Arg Pro Arg Gly Pro Cys Val


          490              500              510              520              530
      *         *         *         *         *         *         *         *         *
GAC GCC GCT GAC CAT CGT CAA CGG TGC GCC GGT CGA CAA GGT CAT CCT
CTG CGG CGA CTG GTA GCA GTT GCC ACG CGG CCA GCT GTT CCA GTA GGA
Asp His His Asp His Arg Gln Arg Cys Ala Gly Arg Gln Gly His Pro


          540              550              560              570
 *         *         *         *         *         *         *         *         *
CGC CGA CAC CAC GGT CCA CCT CAC CGG CGT CTC CGT CAA CGC CGG TGG
GCG GCT GTG GTG CCA GGT GGA GTG GCC GCA GAG GCA GTT GCG GCC ACC
Arg Arg His His Gly Pro Pro His Arg Arg Leu Arg Gln Arg Arg Trp


   580              590              600              610              620
 *         *         *         *         *         *         *         *         *
CAG CCC GGC CAC CGT CAC CGC AGG ACC CGG CGC GAC CCT CGA CGC CAT
GTC GGG CCG GTG GCA GTG GCG TCC TGG GCC GCG CTG GGA GCT GCG GTA
Gln Pro Gly His Arg His Arg Arg Thr Arg Arg Asp Pro Arg Arg His
```

FIG. 2-3

EP 0 426 768 B1

EP 0 426 768 B1

```
           630                 640                  650                 660              670
            *           *        *          *        *         *         *         *      *
CAC CAC CGC ACT GCA GGC ACA GGG CCT CGG GTT CGC GAA CTG CCG GCG
GTG GTG GCG TGA CGT CCG TGT CCC GGA GCC CAA GCG CTT GAC GGC CGC
His His Arg Thr Ala Gly Thr Gly Pro Arg Val Arg Glu Leu Pro His


           680                 690                  700                 710              720
    *        *          *        *          *        *         *         *        *       *
CCC GGT GTG TTG ACC ATC GCC GGC TGC CTC GCC GTC GAC GCT CAC GGT
GGG CCA CAC AAC TGG TAG CGG CCG ACG GAG CGG CAG CTG CGA GTG CCA
Pro Gly Val Leu Thr Ile Ala Gly Cys Leu Ala Val Asp Ala His Gly


           730                 740                  750                 760                770
      *       *          *         *        *         *        *         *         *
GCA GCG CTC CCC GCC GAA GGC GAA GCA CAC GTT CCC GGA CAG ACT TTC
CGT CGC GAG GGG CGG CTT CCG GTY CGT GTG CAR GGG CCT GTC TGA ARG
Ala Ala Leu Pro Ala Glu Gly Glu Ala His Val Pro Gly Gln Thr Phe


           780                 790                  800                 810
    *        *           *        *          *         *        *          *         *
GGC TCA CTC TCC AAC CTC GTC ACC TCC CTG ACC GCA GTG GTC TGG AAC
CCG AGT GAG AGG TTG GAG CAG TGG AGG GAC TGG CGT CAC CAG ACC TTG
Gly Ser Leu Ser Asn Leu Val Thr Ser Leu Thr Ala Val Val Trp Asn
```

FIG. 2-4

EP 0 426 768 B1

```
          820                   830                  840                 850                 860
           *             *       *         *          *            *      *          *        *
          GGC AGT GAG TAC GCG CTC GAG ACG TAC GCG CGT AGC GAT GCA GCG ATC
          CCG TCA CTC ATG CGC GAG CTC TGC ATG CGC GCA TCG CTA CGT CGC TAG
          Gly Ser Glu Tyr Ala Leu Glu Thr Tyr Ala Arg Ser Asp Ala Ala Ile


                  870                  880                 890                 900
            *      *          *         *           *       *          *       *        *
          AAG CCG CTG CTG ACT CAC CTC GGA CGC ACC TTC CTC ACC TCC GTG ACC
          TTC GGC GAC GAC TGA GTG GAG CCT GCG TGG AAG GAG TGG AGG CAC TGG
          Lys Pro Leu Leu Thr His Leu Gly Arg Thr Phe Leu Thr Ser Val Thr


                 920                  930                 940                 950                 960
           *      *         *          *          *        *           *      *         *        *
          TTG CAG GCC GCT CCC AAC TAC CGC ATG CGC TGC GTC AGC CAC ACC GAC
          AAC GTC CGG CGA GGG TTG ATG GCG TAC GCG ACG CAG TCG GTG TGG CTG
          Leu Gln Ala Ala Pro Asn Tyr Arg Met Arg Cys Val Ser His Thr Asp


                 970                  980                 990                1000                1010
           *      *          *         *          *        *           *      *          *
          ATC GGT TGG CAG GAA CTC TTC GGC GCC CGC GGA CGC TCC GGA CGC ACC
          TAG CCA ACC GTC CTT GAG AAG CCG CGG GCG CCT CGC AGG CCT GCG TGG
          Ile Gly Trp Gln Glu Leu Phe Gly His Arg Gly Ala Ser Gly Arg Thr
```

FIG. 2-5

```
            1020            1030            1040            1050
 *      *        *        *      *        *        *      *        *
TTC GAG AAG TTC GTC CGC GAA AAC GGT CGC GCA GAA GCA ATC TGG TAC
AAG CTC TTC AAG CAG GCG CTT TTG CCA GCG CGT CTT CGT TAG ACC ATG
Phe Glu Lys Phe Val Arg Glu Asn Gly Arg Ala Glu Ala Ile Trp Tyr


1060            1070            1080            1090            1100
 *        *        *      *        *        *      *        *        *
CCC TTC ACC GAA CGC CCG TGG ATG AAG GTG TGG TCA CTT GCC CCC ACC
GGG AAG TGG CTT GCG GGC ACC TAC TTC CAC ACC AGT GAA CGG GGG TGG
Pro Phe Thr Glu Arg Pro Trp Met Lys Val Trp Ser Leu Ala Pro Thr


 1110            1120            1130            1140            1150
 *        *        *        *        *      *        *        *      *
AAG CGG CCG TTC TCG CGT GAG GTG ACC GGG CCC TAC AAC TAC ATC TTC
TTC GCC GGC AAG AGC GCA CTC CAC TGG CCC GGG ATG TTG ATG TAG AAG
Lys Arg Pro Phe Ser Arg Glu Val Thr Gly Pro Tyr Asn Tyr Ile Phe


    1160            1170            1180            1190            1200
 *        *        *        *        *        *        *      *        *
TCC GAC AAC CTC CCG GAG CCG GTC ACC GAC ATG ATC GGG CAG ATC AAC
AGG CTG TTG GAG GGC CTC GGC CAG TGG CTG TAC TAG CCC GTC TAG TTG
Ser Asp Asn Leu Pro Glu Pro Val Thr Asp Met Ile Gly Gln Ile Asn
```

FIG. 2-6

EP 0 426 768 B1

```
       1210            1220            1230            1240            1250
   *       *        *       *       *       *       *       *       *
GCC GGA AAC CCG GGC ATC GCA CCG GCA TTC AGG CAG ATC ATG TAC GCC
CGG CCT TTG GGC CCG TAG CGT GGC CGT AAG TCC GTC TAG TAC ATG CGG
Ala Gly Asn Pro Gly Ile Ala Pro Ala Phe Arg Gln Ile Met Tyr Ala


       1260            1270            1280            1290
   *       *        *       *       *       *       *       *       *
ACC ACC GTT GCC GGA CTC GCC GCG ACC TTC TCC AAC GAC CTG TGG GGA
TGG TGG CAA CGG CCT GAG CGG CGC TGG AAG AGG TTG CTG GAC ACC CCT
Thr Thr Val Ala Gly Leu Ala Ala Thr Phe Ser Asn Asp Leu Trp Gly


 1300            1310            1320            1330            1340
   *       *        *       *       *       *       *       *       *
TGG TCC AAG GAC GTC CAG TTC TAC ATC CGG GCC ACC ACC CTT CGT CTG
ACC AGG TTC CTG CAG GTC AAG ATG TAG GCC CGG TGG TGG GAA GCA GAC
Trp Ser Lys Asp Val Gln Phe Tyr Ile Arg Ala Thr Thr Leu Arg Leu


   1350            1360            1370            1380            1390
   *       *        *       *       *       *       *       *       *
ACC GAG GGC GGC GGA GCC CTG ATC ACC TCC CGC GCC AAC ATC GGC CAG
TGG CTC CCG CCG CCT CGG CAG TAG TGG AGG GAG CGG TTG TAG CCG GTC
Thr Glu Gly Gly Gly Ala Val Ile Thr Ser Arg Ala Asn Ile Gly Gln
```

FIG. 2-7

EP 0 426 768 B1

EP 0 426 768 B1

```
          1400          1410          1420          1430          1440
 *         *       *     *       *     *       *     *       *     *
GTC ATC CAC GAC TTC ACC CAG TGG TTC AAC GGC CGC ATG GAG TAC TAC
CAG TAG GTG CTG AAG TGG GTC ACC AAG TTG CCG GCG TAC CTC ATG ATG
Val Ile His Asp Phe Thr Gln Trp Phe Asn Gly Arg Met Glu Tyr Tyr


          1450          1460          1470          1480          1490
 *         *       *     *       *     *       *     *       *
CGC TCT ATC GGA CAG TTC CCC CTC AAC GGC CCC GTC GAA ATC GCT TGC
GCG AGA TAG CCT GTC AAG GGG GAG TTG CCG GGG CAG CTT TAG GCA ACG
Arg Ser Ile Gly Gln Phe Pro Leu Asn Gly Pro Val Glu Ile Arg Cys


          1500          1510          1520          1530
 *         *       *     *       *     *       *     *       *
TGC GGC CTC GAT CAA CCC TCG GAC GTC GAG GTC GAC TCG GCA GGC CCC
ACG CCG GAG CTA GTT GGG AGC CTG CAG CTC CAG CTG AGC CGT CCG GGG
Cys Gly Leu Asp Gln Pro Ser Asp Val Glu Val Asp Ser Ala Gly Pro


 1540         1550          1560          1570          1580
 *         *       *     *       *     *       *     *       *
CCC ACC ATC TCG GCC ATG CGC CCG CGC CCA GAC CAT CCG GAA TGG GAC
GGG TGG TAG AGC CGG TAC GCG GGC GCG GGT CTG GTA GGC CTT ACC CTG
Pro Thr Ile Ser Ala Met Arg Pro Arg Pro Asp His Pro Glu Trp Asp
```

FIG. 2-8

EP 0 426 768 B1

```
      1590          1600          1610          1620          1630
       *        *     *        *     *        *     *        *     *
ACC GCC ATC TGG CTC AAC GTC CTC GGC GTC CCC GGA ACC CCC GGC ATG
TGG CGG TAG ACC GAG TTG CAG GAG CCG CAG GGG CCT TGG GGG CCG TAC
Thr Ala Ile Trp Leu Asn Val Leu Gly Val Pro Gly Thr Pro Gly Met


      1640          1650          1660          1670          1680
 *        *     *        *     *        *     *        *     *        *
TTC GCG TTC TAC CGC GAA ATG GAA CAG TGG ATG CCT AAT CAC TAC AAC
AAG CGC AAG ATG GCG CTT TAC CTT GTC ACC TAC GGA TTA GTG ATG TTG
Phe Ala Phe Tyr Arg Glu Met Glu Gln Trp Met Pro Asn His Tyr Asn


      1690          1700          1710          1720          1730
    *        *     *        *     *        *     *        *     *
AAC AAC GAC GCC ACC TTC CGC CCA GAA TGG TCC AAG GGC TGG CGT TCG
TTG TTG CTG CGG TGG AAG GCG GGT CTT ACC AGG TTC CCG ACC GCA AGC
Asn Asn Asp Ala Thr Phe Arg Pro Glu Trp Ser Lys Gly Trp Arg Ser


      1740          1750          1760          1770
 *        *     *        *     *        *     *        *     *
GCC CCG ACA AGC CGT ACA CCG ACG CCC CGA TCA TCA CCC AGG GCC TCC
CGG GGC TGT TCG GCA TGT GGC TGC GGG GCT AGT AGT GGG TCC CGG AGG
Ala Pro Thr Ser Arg Thr Pro Thr Pro Arg Ser Ser Pro Arg Ala Ser
```

FIG. 2-9

```
 1780           1790           1800           1810           1820
  *        *        *        *        *        *        *        *        *        *
CGC AGA CCT ACC GCG ACG GCG TCC CAT CGA GCG ACA ACT GGG ACA CCG
GCG TCT GGA TGG CGC TGC CAC AGG GTA GCT CGC TGT TGA CCC TGT GGC
Arg Arg Pro Thr Ala Thr Ala Ser His Arg Ala Thr Thr Gly Thr Pro


  1830           1840           1850           1860           1870
   *        *        *        *        *        *        *        *        *
CCA ACG CCG CAT ACA ACG CGT TGG ATC CGC ACA AGG TCT TCA GCA ACA
GGT TGC GGC GTA TGT TGC GCA ACC TAG GCG TGT TCC AGA AGT CGT TGT
Pro Thr Pro His Thr Thr Arg Trp Ile Arg Thr Arg Ser Ser Ala Thr


   1880           1890           1900           1910           1920
  *        *        *        *        *        *        *        *        *        *
CCT TCC TGG ACC AGT TGC TGC CCT GAG GTC CAA GCA CCA GGC ACG CAC
GGA AGG ACC TGG TCA ACG ACG GGA CTC CAG GTT CGT GGT CCG TGC GTG
Pro Ser Trp Thr Ser Cys Cys Pro Glu Val Gln Ala Pro Gly Thr His


      1930           1940           1950           1960           1970
   *        *        *        *        *        *        *        *        *
TCT GCC AAC TCG ACG GTC GTC GCC ACT CCC CTA CTT TCG GGA GTG CGG
AGA CGG TTG AGC TGC CAG CAG CGG TGA GGG GAT GAA AGC CCT CAC GCC
Ser Ala Asn Ser Thr Val Val Ala Thr Pro Leu Leu Ser Gly Val Arg
```

FIG. 2-10

EP 0 426 768 B1

```
           1980          1990          2000          2010
  *      *      *      *      *      *      *      *      *      *
CCG CCG TCG TGC GTT GCA GTG AGC GGG ATG AAG GAG TCC CCC CGC ATC
GGC GGC AGC ACG CAA CGT CAC TCG CCC TAC TTC CTC AGG GGG GCG TAG
Pro Pro Ser Cys Val Ala Val Ser Gly Met Lys Glu Ser Pro Arg Ile


2020          2030
  *      *      *
CCG ACC AGG TAA C
GGC TGG TCC ATT G                                              FIG. 2-11
Pro Thr Arg End


     2040        2050        2060        2070        2080        2090
   *      *      *      *      *      *      *      *      *      *      *      *
TGACTCAATC GCGATCAGGA TGGATCGCCT CGTGCATACT TTCCACGGCC CTGGCATGAG
ACTGAGTTAG CGCTAGTCCT ACCTAGCGGA GCACGTATGA AAGGTGCCGG GACCGTACTC


     2100        2110        2120        2130        2140        2150
   *      *      *      *      *      *      *      *      *      *      *      *
TTGAGAGCTC GGACCGAGCC GACCGAATCG TGGCGCTGTC GCTGCGCGCC CCCGGCTCGG
AACTCTCGAG CCTGGCTCGG CTGGCTTAGC ACCGCGACAG CGACGCGCGG GGGCCGAGCC


     2160        2170        2180        2190        2200        2210
   *      *      *      *      *      *      *      *      *      *      *      *
ACGACGACGA GTCCTCTCCG GGGTTCGACG CGCCACCAGC GGGATGCTCG GAGGAGCCGG
TGCTGCTGCT CAGGAGAGGC CCCAAGCTGC GCGGTGGTCG CCCTACGAGC CTCCTCGGCC
```

EP 0 426 768 B1

EP 0 426 768 B1

```
       2220          2230          2240          2250          2260          2270
  *         *    *         *    *         *    *         *    *         *    *         *
TGCGCTCTTC CCTCTCCGCG CGGGCGCGCA ACAACGACTC GGACTTCGGG GTATTTCGCA
ACGCGAGAAG GGAGAGGCGC GCCCGCGCGT TGTTGCTGAG CCTGAAGCCC CATAAAGCGT

       2280          2290          2300          2310          2320          2330
  *         *    *         *    *         *    *         *    *         *    *         *
TCGGAGCATT GTCCCAGATT CATCTCCCAT GACTCACATG GAGCGCTCTC ACCCCCGGTG
AGCCTCGTAA CAGGGTCTAA GTAGAGGGTA CTGAGTGTAC CTCGCGAGAG TGGGGGCCAC


       *
ACCAGGCC
TGGTCCGG
```

FIG. 2-12